# FASCICULE DE BREVET EUROPEEN

(11) **EP 0 556 098 B1**
(45) Date de publication et mention de la délivrance du brevet: **14.10.1998**
(21) Numéro de dépôt: 93400281.7
(22) Date de dépôt: 04.02.1993
(51) Int. Cl.: A61J 3/00, A61M 1/16

(54) **Dispositif et procédé pour la préparation d'une solution à usage médical**
Vorrichtung und Verfahren zur Herstellung einer medizinischen Lösung
Device and method for preparation of a medical solution

(30) Priorité: 14.02.1992 FR 9201851
(43) Date de publication de la demande: 18.08.1993
(73) Titulaire: Lascombes, Jean-Jacques, F-31000 Toulouse (FR)
(72) Inventeur: Lascombes, Jean-Jacques, F-31000 Toulouse (FR)
(74) Mandataire: Morelle, Guy Georges Alain

(56) Documents cités:
- EP-A- 0 278 100
- EP-A- 0 401 130
- EP-A- 0 439 793
- EP-A- 0 443 324
- WO-A-86/00797
- WO-A-92/05814

## Description

La présente invention concerne un procédé et un dispositif pour la préparation d'une solution à usage médical par dissolution de plusieurs substances sous forme de poudre dans un fluide porteur et elle brise plus particulièrement, mais de manière non spécifique, un procédé et un dispositif pour la préparation d'une solution pour dialyse.

Pour effectuer une séance d'hémodialyse, il est nécessaire de disposer d'un appareil connu sous le nom de générateur. Dans ce genre d'appareil, on utilise un fluide porteur qui est généralement de l'eau purifiée, la préparation du dialysat étant réalisée à partir de concentrés sous forme liquide ou sous forme de poudre, dilués dans ce fluide porteur.

L'emploi des générateurs de ce type nécessite ainsi le transport vers les lieux de traitement de volumes importants de produits préalablement préparés et leur stockage sur les lieux en question, ces contraintes étant à l'origine de coûts d'exploitation élevés.

On connaît par ailleurs, par le EP-A-0 278 100 et par le EP-A-0 401 130, des dispositifs qui réduisent quelque peu les inconvénients précités en permettant une préparation en ligne de certains composants présentés sous forme de poudre. Ces dispositifs comprennent ainsi une conduite principale ayant une première extrémité reliée à une source d'eau et une seconde extrémité pour délivrer une solution de dialyse, et plusieurs conduites secondaires reliées en dérivation à la conduite principale et sur chacune desquelles est monté un réservoir ou cartouche contenant un produit pulvérulent qui, après dissolution, est mélangé à l'eau circulant dans la conduite principale.

Ces dispositifs requièrent dans la pratique l'emploi d'un concentré sous forme liquide qui contient les autres substances nécessaires au traitement. Ainsi, il est nécessaire de préparer à l'avance et de conserver dans un conteneur ces autres substances dont les quantités et les niveaux de concentration respectifs ne peuvent évidemment être modifiés pour les adapter éventuellement aux besoins particuliers des patients au cours du traitement.

Le procédé de l'invention et le dispositif qui permet de le mettre en oeuvre, autorisent notamment la suppression de cette phase de préparation.

Il est possible en effet de préparer en ligne le dialysat, en assurant un contrôle permanent et une modification éventuelle instantanée de la concentration des différents solutés entrant dans sa composition.

De manière générale, l'invention a ainsi pour objet un procédé de préparation d'une solution à usage médical par dissolution dans un fluide porteur de plusieurs substances sous forme de poudre, ceci au moyen d'un dispositif dans lequel lesdites substances sont contenues dans des cellules et qui comprend une première conduite communiquant avec une entrée desdites cellules pour introduire ledit fluide porteur dans lesdites cellules afin de produire des solutés dans lesdites cellules, une seconde conduite communiquant avec une sortie desdites cellules pour amener lesdits solutés produits dans lesdites cellules vers un point de mélange situé en amont d'un lieu d'utilisation, un moyen de mesure pour mesurer la concentration desdits solutés et un moyen de régulation de débit pour modifier la concentration desdits solutés en réponse aux informations fournies par au moins ledit moyen de mesure, ce procédé étant remarquable en qu'il consiste à dissoudre l'ensemble des substances nécessaires à la préparation de ladite solution dans lesdites cellules, chaque cellule contenant au moins une desdites substances sous forme de poudre.

Le procédé tel que défini ci-dessus trouve une application particulièrement intéressante pour la préparation de manière extemporanée et en continu d'une solution pour dialyse par dissolution dans de l'eau de plusieurs substances contenues sous forme de poudre dans des cartouches, ce procédé consistant à dissoudre l'ensemble des substances nécessaires à la préparation de ladite solution de dialyse dans lesdites cartouches, chaque cartouche contenant sous forme de poudre au moins un des sels nécessaires à la préparation d'un dialysat tel que chlorure de sodium, chlorure de potassium, chlorure de calcium, chlorure de magnésium et bicarbonate de sodium.

L'invention a également pour objet un dispositif permettant la mise en oeuvre de ce procédé.

Ce dispositif est remarquable notamment par le fait que chaque cellule ou cartouche comporte un moyen de diffusion et de circulation dudit fluide porteur ou de l'eau dans lesdites cellules ou cartouches.

Ce moyen, qui permet d'introduire de l'eau dans le volume de poudre contenu dans les cartouches et de faciliter l'obtention de solutions concentrées, est constitué par au moins une fibre membranaire semi-perméable, disposée sensiblement selon un axe longitudinal desdites cartouches entre l'entrée et la sortie de ces dernières.

Bien évidemment, ces cartouches sont livrées sous forme sèche et stérile, ce qui élimine tout risque de contamination. De plus, la forme poudre utilisée avec ces cartouches est une forme très stable d'un point de vue physico-chimique ainsi que bactériologique.

D'autres caractéristiques et avantages de la présente invention ressortiront à la lecture de la description qui suit d'un exemple de réalisation d'un dispositif pour la préparation d'une solution pour dialyse conforme aux premiers enseignements ci-dessus donnés, cet exemple étant donné à titre simplement d'illustration et sans qu'aucune interprétation restrictive de la protection recherchée puisse en être tirée.

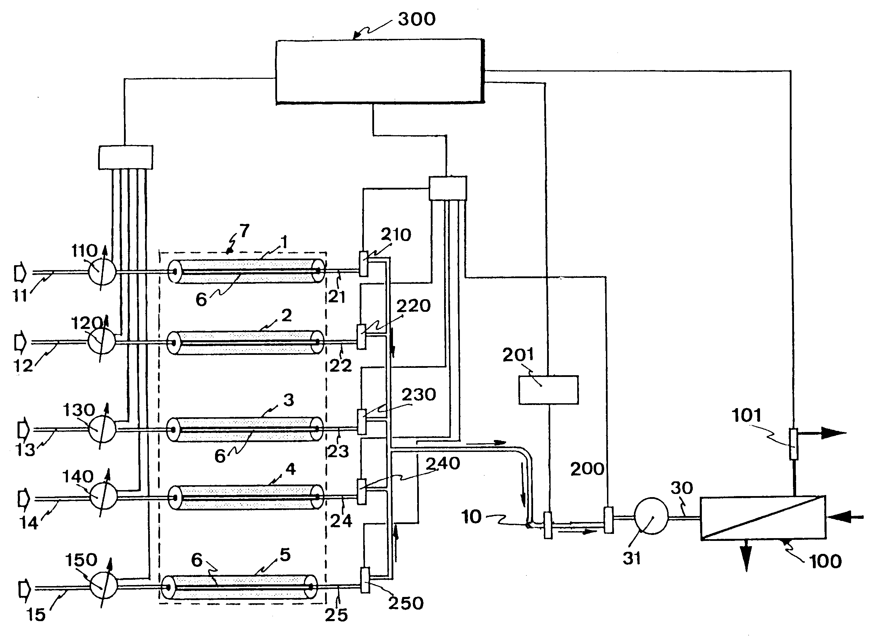

Cette description est accompagnée d'un dessin dont la Figure unique montre le bloc diagramme d'une installation destinée plus particulièrement à la préparation extemporanée et en continu d'une solution pour dialyse.

Cette installation est organisée autour d'un dispositif constitué d'une batterie de cinq cartouches, respectivement référencées de 1 à 5, disposées en parallèle et qui renferment chacune un sel d'une substance nécessaire à la préparation du dialysat : chlorure de sodium, chlorure de potassium, chlorure de calcium, chlorure de magnésium et bicarbonate de sodium.

Ainsi que précisé plus avant, le volume intérieur de chaque cartouche est occupé partiellement par une fibre membranaire semi-perméable 6 disposée sensiblement selon un axe longitudinal desdites cartouches dans le prolongement de premières conduites, respectivement référencées de 11 à 15, dont une première extrémité est reliée à une source d'eau purifiée non réprésentée qui, avantageusement, pourra être la source utilisée pour alimenter le circuit d'eau du générateur de dialyse.

Cette eau pénétre ainsi par une seconde extrémité desdites premières conduites dans chaque cartouche 1-5 et suit le trajet défini par les fibres membranaires semi-perméables. Dans un premier temps, l'eau ainsi véhiculée se diffuse à l'intérieur desdites cartouches par perméation au travers des fibres membranaires, remplit le volume desdites cartouches et se mélange aux substances poudreuses qui enveloppent les fibres. L'eau ainsi chargée en sel suit alors le trajet défini par les fibres membranaires et les solutions concentrées produites dans chaque cartouche vont être dirigées vers un point où elles seront mélangées.

Pour ce faire, en sortie des cartouches 1-5, on trouve des secondes conduites, respectivement référencées 21 à 25, destinées à collecter les différentes solutions concentrées produites pour les véhiculer vers un point de mélange constitué par une troisième conduite 10, qui est raccordée aux circuits fluidiques d'un générateur de dialyse 100, par une conduite 30 munie d'une pompe 31.

L'un des principaux intérêts du dispositif de l'invention est qu'il permet d'adapter la composition du dialysat à chaque cas clinique, ainsi que de manière permanente pendant la séance de dialyse, en fonction des données propres à chaque patient relevées au cours du traitement.

A cet effet, le dispositif comporte des premiers moyens de mesure de la concentration des solutions produites dans chaque cartouche 1-5, et qui sont constitués par des cellules de conductivité disposées sur les secondes conduites 21-25 en sortie desdites cartouches et en amont de la troisième conduite ou conduite de "mélange" 10.

Ces cellules de conductivité, respectivement référencées 210 à 250 sur le dessin annexé, transmettent ainsi les informations qu'elles recueillent à un système 300 de pilotage de l'installation qui commande des pompes à débit variable, respectivement référencées 110 à 150, montées sur les premières conduites 11-15 afin de régler le débit d'arrivée d'eau purifiée dans chaque cartouche.

Selon une variante de réalisation du dispositif de l'invention, non illustrée, ces pompes peuvent être avantageusement montées sur les secondes conduites 21-25 afin de limiter la perte de charge qui peut se produire avec des pompes montées sur les premières conduites 11-15. De plus, avec un tel montage, la régulation du débit est plus précise et constante.

En effet, comme on l'a vu, chaque cartouche du dispositif de l'invention contient un sel et la seule variation du débit d'eau permet la régulation du transfert de sel au travers de la fibre membranaire semi-perméable qui équipe ces cartouches. Cette variation du débit d'eau permet ainsi de modifier la concentration en sel des solutions obtenues.

La concentration des solutions est en outre contrôlée par des seconds moyens de mesure 101 disposés en aval du générateur de dialyse 100, par exemple sur la sortie du dialysat et/ou sur le circuit sanguin du patient, ces seconds moyens de mesure agissant par l'intermédiaire du système d'asservissement 300 sur les pompes de régulation du débit de l'eau 110-150.

Pour chaque sel, le coefficient de transfert par diffusion est déterminé. Ce coefficient permet dès lors de prévoir la concentration en sel à la sortie de chaque cartouche en fonction du débit d'eau utilisé. Ce débit d'eau est ainsi réglé en fonction de la composition de la solution de dialyse obtenue dans la conduite de mélange 10, cette composition étant adaptée à chaque malade à partir notamment des données recueillies au cours de la séance.

Les différentes cartouches, mais également les fibres qu'elles renferment, sont dimensionnées de telle sorte que la concentration en millimoles d'ions par litre dans la solution de dialyse soit située dans les fourchettes suivantes :
- de 135 à 150 mmol pour l'ion sodium,
- de 1 à 3 mmol pour l'ion potassium,
- de 0 à 1,75 mmol pour l'ion calcium,
- de 0,25 à 1 mmol pour l'ion magnésium,
- de 25 à 35 mmol pour l'ion bicarbonate.

On trouvera ci-après deux tableaux indiquant, à titre d'exemple, le volume des différentes cartouches, exprimé en cm³, pour chaque sel entrant dans la composition d'une solution utilisée pour des séances de dialyse d'une durée respectivement de 6 heures et de 8 heures.

**Tableau 1**

| Séance de dialyse d'une durée de 6 heures. | |
|---|---|
| Chlorure de sodium : | 1270,80 |
| Chlorure de potassium : | 184,60 |
| Chlorure de calcium : | 184,60 |
| Chlorure de magnesium : | 276,60 |
| Bicarbonate de sodium : | 1270,90 |

**Tableau 2**

| Séance de dialyse d'une durée de 8 heures. | |
|---|---|
| Chlorure de sodium : | 1694,50 |
| Chlorure de potassium : | 246,10 |
| Chlorure de calcium : | 246,20 |
| Chlorure de magnésium : | 368,80 |
| Bicarbonate de sodium : | 1694,50 |

On ajoutera que le dispositif de l'invention comporte également des moyens pour alimenter la solution de dialyse circulant dans la conduite 10 en produits de supplémentation tels que glucose, acides aminés et oligo-éléments, ces moyens n'étant pas plus amplement décrits car leur réalisation est à la simple portée de l'Homme de l'Art.

Ce dispositif comprend par ailleurs un moyen de mesure du pH de la solution de dialyse, constitué par une cellule 200 montée sur la conduite 10 et reliée à un pH-mètre 201, qui permet ainsi d'ajuster le pH du mélange circulant dans ladite conduite à une valeur physiologique.

Nécessairement, un tel dispositif est équipé de moyens de régulation thermique afin de maintenir la solution de dialyse à une température clinique appropriée, par exemple à une température de 37°C.

Ces moyens de régulation thermique pourront, de manière connue, comprendre par exemple une résistance électrique associée à la conduite de mélange 10.

Il est évident que le dispositif qui vient d'être décrit peut faire l'objet de diverses modifications sans que sa conception subisse de profonds bouleversements. Ainsi, par exemple, les cartouches peuvent être regroupées dans une enceinte 7, représentée en traits interrompus, munie d'une seule entrée d'eau et d'un système de vanne permettant d'alimenter sélectivement en eau l'une ou l'autre desdites cartouches, l'essentiel étant de conserver au dispositif son caractère modulaire qui résulte notamment de la possibilité d'ajouter ou d'enlever des cartouches contenant différentes substances en fonction de la nature de la solution finale que l'on souhaite obtenir.

## Revendications

1. Procédé de préparation d'une solution à usage médical par dissolution dans un fluide porteur de plusieurs substances sous forme de poudre, au moyen d'un dispositif dans lequel lesdites substances sont contenues dans des cellules et qui comprend une première conduite (11-15) communiquant avec une entrée desdites cellules (1-5) pour introduire ledit fluide porteur dans lesdites cellules afin de produire des solutés dans lesdites cellules, une seconde conduite (21-25) communiquant avec une sortie desdites cellules pour amener lesdits solutés produits dans lesdites cellules vers un point de mélange (10) situé en amont d'un lieu d'utilisation (100), un moyen de mesure (210-250) pour mesurer la concentration desdits solutés et un moyen de régulation de débit (110-150) pour modifier la concentration desdits solutés en réponse aux informations fournies par au moins ledit moyen de mesure, ledit procédé étant ***caractérisé en ce qu'***il consiste à dissoudre l'ensemble des substances nécessaires à la préparation de ladite solution dans lesdites cellules, chaque cellule contenant au moins une desdites substances sous forme de poudre.

2. Dispositif permettant la mise en oeuvre du procédé selon la Revendication 1, comprenant une première conduite (11-15) communiquant avec une entrée desdites cellules (1-5) pour introduire ledit fluide porteur dans lesdites cellules afin de produire des solutés dans lesdites cellules, une seconde conduite (21-25) communiquant avec une sortie desdites cellules pour amener lesdits solutés produits dans lesdites cellules vers un point de mélange (10) situé en amont d'un lieu d'utilisation (100), un moyen de mesure (210-250) pour mesurer la concentration desdits solutés et un moyen de régulation de débit (110-150) pour modifier la concentration desdits solutés en réponse aux informations fournies par au moins ledit moyen de mesure, ***caractérisé en ce que*** chaque dite cellule comporte un moyen de diffusion et de circulation dudit fluide porteur dans lesdites cellules.

3. Dispositif selon la Revendication 2, ***caractérisé en ce que*** ledit moyen de mesure (210-250) est monté en amont dudit point de mélange (10).

4. Dispositif selon la Revendication 2, ***caractérisé en ce qu'***il comporte en outre un second moyen de mesure (101) monté en aval dudit lieu d'utilisation (100).

5. Procédé de préparation extemporanée et en continu d'une solution pour dialyse par dissolution dans de l'eau de plusieurs substances sous forme de poudre, au moyen d'un dispositif dans lequel lesdites substances sont contenues dans des cartouches et qui comprend une première conduite (11-15) communiquant avec une entrée desdites cartouches (1-5) pour introduire de l'eau dans lesdites cartouches afin de produire des solutés dans lesdites cartouches, une seconde conduite (21-25) communiquant avec une sortie desdites cartouches pour amener lesdits solutés vers un point de mélange (10) situé en amont d'un circuit de dialyse (100), un moyen de mesure (210-250) pour mesurer la concentration desdits solutés et un moyen de régulation de débit (110-150) pour modifier la concentration desdits solutés en réponse aux informations fournies par au moins ledit moyen de mesure, ledit procédé étant ***caractérisé en ce qu'***il consiste à dissoudre l'ensemble des substances nécessaires à la préparation de ladite solution de dialyse dans lesdites cartouches, chaque cartouche contenant sous forme de poudre au moins un des sels nécessaires à la préparation d'un dialysat tel que chlorure de sodium, chlorure de potassium, chlorure de calcium, chlorure de magnésium et bicarbonate de sodium.

6. Dispositif permettant la mise en oeuvre du procédé selon la Revendication 5, comprenant une première conduite (11-15) communiquant avec une entrée desdites cartouches (1-5) pour introduire de l'eau dans lesdites cartouches afin de produire des solutés dans lesdites cartouches, une seconde conduite (21-25) communiquant avec une sortie desdites cartouches pour amener lesdits solutés vers un point de mélange (10) situé en amont d'un circuit de dialyse (100), un moyen de mesure (210-250) pour mesurer la concentration desdits solutés et un moyen de régulation de débit (110-150) pour modifier la concentration desdits solutés en réponse aux informations fournies par au moins ledit moyen de mesure, ***caractérisé en ce que*** ledit moyen de mesure (210-250) est monté sur ladite seconde conduite (21-25) en amont dudit point de mélange (10), pour mesurer la concentration desdits solutés en sortie desdites cartouches.

7. Dispositif selon la Revendication 6, ***caractérisé en ce qu'il*** comporte en outre un second moyen de mesure (101) monté en aval dudit circuit de dialyse (100).

8. Dispositif selon l'une quelconque des Revendications 6 et 7, ***caractérisé en ce que*** ledit moyen de régulation de débit (110-150) est monté sur ladite première conduite (11-15) en amont desdites cartouches (1-5), pour régler le débit de l'eau afin de modifier la concentration desdits solutés en réponse aux informations fournies par lesdits premier et/ou second moyens de mesure.

9. Dispositif selon l'une quelconque des Revendications 6 et 7, ***caractérisé en ce que*** ledit moyen de régulation de débit (110-150) est monté sur ladite seconde conduite (21-25).

10. Dispositif selon la Revendication 6, ***caractérisé en ce que*** chaque dite cartouche comporte un moyen (6) de diffusion et de circulation de l'eau entre lesdites entrée et sortie desdites cartouches.

11. Dispositif selon la Revendication 10, ***caractérisé en ce que*** ledit moyen est disposé sensiblement selon un axe longitudinal desdites cartouches.

12. Dispositif selon la Revendication 10 ou 11, ***caractérisé en ce que*** ledit moyen est constitué par au moins une fibre membranaire semi-perméable (6).

13. Dispositif selon la Revendication 12, ***caractérisé en ce que*** lesdites cartouches et fibres sont dimensionnées de telle sorte que la concentration en millimoles d'ions par litre dans la solution de dialyse obtenue soit située dans les fourchettes suivantes :
- de 135 à 150 mmol pour l'ion sodium,
- de 1 à 3 mmol pour l'ion potassium,
- de 0 à 1,75 mmol pour l'ion calcium,
- de 0,25 à 1 mmol pour l'ion magnésium,
- de 25 à 35 mmol pour l'ion bicarbonate.

14. Dispositif selon la Revendication 6, ***caractérisé en ce que*** ledit point de mélange est constitué par une troisième conduite (10) montée en entrée du susdit circuit de dialyse (100).

15. Dispositif selon la Revendication 6 ou 7, ***caractérisé en ce que*** lesdits moyens de mesure comprennent des cellules de conductivité (210-250).

16. Dispositif selon la Revendication 8 ou 9, ***caractérisé en ce que*** ledit moyen de régulation de débit comprend des pompes à débit variable (110-150).

17. Dispositif selon la Revendication 14, ***caractérisé en ce qu'***il comprend un moyen de mesure (200-201) du pH du mélange obtenu dans ladite troisième conduite.

18. Dispositif selon la Revendication 14, ***caractérisé en ce qu'***il comprend des moyens pour introduire dans ladite troisième conduite (10) des produits de supplémentation tels que glucose, acides aminés et oligo-éléments.

19. Dispositif selon l'une quelconque des Revendications 6 à 18, ***caractérisé en ce qu'***il comprend des moyens de régulation thermique pour maintenir la solution de dialyse à une température déterminée.

20. Dispositif selon l'une quelconque des Revendications précédentes ***caractérisé en ce que*** lesdites cartouches sont logées dans une enceinte (7).

## Claims

1. Method of preparing a solution for medical use by dissolving a plurality of substances, in powder form, in a carrier fluid by means of an apparatus in which said substances are contained in cells, and which apparatus comprises a first line (11-15) communicating with an inlet of said cells (1-5) to introduce said carrier fluid into said cells in order to produce solutes in said cells, a second line (21-25) communicating with an outlet of said cells to convey said solutes produced in said cells towards a mixing point (10) situated upstream of a place of use (100), a measuring means (210-250) for measuring the concentration of said solutes and a flow regulating means (110-150) to modify the concentration of said solutes in response to the information provided by at least said measuring means, said method being characterised in that it comprises dissolving all of the substances necessary for the preparation of said solution in said cells, each cell containing at least one of said substances in powder form.

2. Apparatus permitting the method according to claim 1 to be accomplished, comprising a first line (11-15) communicating with an inlet of said cells (1-5) to introduce said carrier fluid into said cells in order to produce solutes in said cells, a second line (21-25) communicating with an outlet of said cells to convey said solutes produced in said cells towards a mixing point (10) situated upstream of a place of use (100), a measuring means (210-250) for measuring the concentration of said solutes and a flow regulating means (110-150) to modify the concentration of said solutes in response to the information provided by at least said measuring means, characterised in that each said cell comprises a means for diffusing and circulating said carrier fluid in said cells.

3. Apparatus according to claim 2, characterised in that said measuring means (210-250) is mounted upstream of said mixing point (10).

4. Apparatus according to claim 2, characterised in that it also includes a second measuring means (101) mounted downstream of said place of use (100).

5. Method of extemporaneously and continuously preparing a solution for dialysis by dissolving in water a plurality of substances, in powder form, by means of an apparatus in which said substances are contained in cartridges, and which apparatus comprises a first line (11 -15) communicating with an inlet of said cartridges (1-5) to introduce water into said cartridges in order to produce solutes in said cartridges, a second line (21-25) communicating with an outlet of said cartridges to convey said solutes towards a mixing point (10) situated upstream of a dialysis circuit (100), a measuring means (210-250) for measuring the concentration of said solutes and a flow regulating means (110-150) to modify the concentration of said solutes in response to the information provided by at least said measuring means, said method being characterised in that it comprises dissolving all of the substances necessary for the preparation of said dialysis solution in said cartridges, each cartridge containing in powder form at least one of the salts necessary for the preparation of a dialysate such as sodium chloride, potassium chloride, calcium chloride, magnesium chloride and sodium bicarbonate.

6. Apparatus permitting the method according to claim 5 to be accomplished, comprising a first line (11-15) communicating with an inlet of said cartridges (1-5) to introduce water into said cartridges in order to produce solutes in said cartridges, a second line (21-25) communicating with an outlet of said cartridges to convey said solutes towards a mixing point (10) situated upstream of a dialysis circuit (100), a measuring means (210-250) for measuring the concentration of said solutes and a flow regulating means (110-150) to modify the concentration of said solutes in response to the information provided by at least said measuring means, characterised in that said measuring means (210-250) is mounted on said second line (21-25) upstream of said mixing point (10) to measure the concentration of said solutes at the outlet of said cartridges.

7. Apparatus according to claim 6, characterised in that it also includes a second measuring means (101) mounted downstream of said dialysis circuit (100).

8. Apparatus according to any one of claims 6 and 7, characterised in that said flow regulating means (110-150) is mounted on said first line (11-15) upstream of said cartridges (1-5) to regulate the flow of the water in order to modify the concentration of said solutes in response to the information provided by said first and/or second measuring means.

9. Apparatus according to any one of claims 6 and 7, characterised in that said flow regulating means (110-150) is mounted on said second line (21-25).

10. Apparatus according to claim 6, characterised in that each said cartridge comprises a means (6) for diffusing and circulating the water between said inlet and outlet of said cartridges.

11. Apparatus according to claim 10, characterised in that said means is disposed substantially along a longitudinal axis of said cartridges.

12. Apparatus according to claim 10 or 11, characterised in that said means is formed by at least one membranous, semi-permeable fibre (6).

13. Apparatus according to claim 12, characterised in that said cartridges and fibres are so dimensioned that the concentration in millimols of ions per litre in the dialysis solution obtained is within the following ranges:
- from 135 to 150 mmol for the sodium ion,
- from 1 to 3 mmol for the potassium ion.
- from 0 to 1.75 mmol for the calcium ion,
- from 0.25 to 1 mmol for the magnesium ion, and
- from 25 to 35 mmol for the bicarbonate ion.

14. Apparatus according to claim 6, characterised in that said mixing point is formed by a third line (10) mounted at the inlet of the aforementioned dialysis circuit (100).

15. Apparatus according to claim 6 or 7, characterised in that said measuring means comprise conductivity cells (210-250).

16. Apparatus according to claim 8 or 9, characterised in that said flow regulating means comprises variable delivery pumps(110-150).

17. Apparatus according to claim 14, characterised in that it comprises a means (200-201) for measuring the pH of the mixture obtained in said third line.

18. Apparatus according to claim 14, characterised in that it comprises means for introducing into said third line (10) supplementary products such as glucose, amino acids and trace elements.

19. Apparatus according to any one of claims 6 to 18, characterised in that it comprises heat regulating means to maintain the dialysis solution at a specific temperature.

20. Apparatus according to any one of the preceding claims, characterised in that said cartridges are accommodated in a chamber (7).

## Patentansprüche

1. Verfahren zur Herstellung einer medizinisch verwendbaren Lösung durch Lösen mehrerer pulverförmiger Substanzen in einer Trägerflüssigkeit mit Hilfe einer Vorrichtung, in der die Substanzen in Zellen enthalten sind und die eine erste Leitung (11-15), die mit einem Eingang der Zellen (1-5) verbunden ist, um diesen die Trägerflüssigkeit zuzuführen und in ihnen Lösungen zu erzeugen, eine zweite Leitung (21-25), die mit einem Ausgang der Zellen verbunden ist, um die darin erzeugten Lösungen einem oberhalb einer Verwendungsstelle (100) angeordneten Mischungspunkt (10) zuzuführen, ein Meßgerät (210-250) zur Messung der Konzentration der Lösungen und einen Durchsatzregler (110-150) zur Einstellung der Konzentration der Lösungen entsprechend den zumindest vom Meßgerät gelieferten Daten umfaßt, wobei das Verfahren dadurch **gekennzeichnet** ist, daß es darin besteht, daß sämtliche für die Herstellung der Lösung benötigten Substanzen in den Zellen gelöst werden, von denen jede wenigstens eine der pulverförmigen Substanzen enthält.

2. Vorrichtung zur Durchführung des Verfahrens nach Anspruch 1, die eine erste Leitung (11-15), die mit einem Eingang der Zellen (1-5) verbunden ist, um diesen die Trägerflüssigkeit zuzuführen und in ihnen Lösungen zu erzeugen, eine zweite Leitung (21-25), die mit einem Ausgang der Zellen verbunden ist, um die darin erzeugten Lösungen einem oberhalb einer Verwendungsstelle (100) angeordneten Mischungspunkt (10) zuzuführen, ein Meßgerät (210-250) zur Messung der Konzentration der Lösungen und einen Durchsatzregler (110-150) zur Einstellung der Konzentration der Lösungen entsprechend den zumindest vom Meßgerät gelieferten Daten umfaßt, dadurch **gekennzeichnet,** daß jede Patrone ein Mittel zur Verteilung und Umwälzung der Trägerflüssigkeit in den Zellen umfaßt.

3. Vorrichtung nach Anspruch 2, dadurch **gekennzeichnet,** daß das Meßgerät (210-250) oberhalb des Mischungspunktes (10) angebracht ist.

4. Vorrichtung nach Anspruch 2, dadurch **gekennzeichnet,** daß sie außerdem noch ein zweites unterhalb der Verwendungsstelle (100) angebrachtes Meßgerät (101) umfaßt.

5. Verfahren zur sofortigen und kontinuierlichen Herstellung einer Dialyselösung durch Lösen mehrerer pulverförmiger Substanzen in Wasser mit Hilfe einer Vorrichtung, in der die Substanzen in Patronen enthalten sind und die eine erste Leitung (11-15), die mit einem Eingang der Patronen (1-5) verbunden ist, um diesen Wassser zuzuführen und in ihnen Lösungen zu erzeugen, eine zweite Leitung (21-25), die mit einem Ausgang der Patronen verbunden ist, um die Lösungen einem oberhalb eines Dialysekreislaufs (100) angeordneten Mischungspunkt (10) zuzuführen, ein Meßgerät (210-250) zur Messung der Konzentration der Lösungen und einen Durchsatzregler (110-150) zur Einstellung der Konzentration der Lösungen entsprechend den zumindest vom Meßgerät gelieferten Daten umfaßt, wobei das Verfahren dadurch **gekennzeichnet** ist, daß es darin besteht, daß sämtliche für die Herstellung der Dialyselösung benötigten Substanzen in den Patronen gelöst werden, von denen jede wenigstens eines der für die Herstellung eines Dialysats erforderlichen Salze wie Natrium-, Kalium-, Calcium- und Mangesiumchlorid sowie Natriumbicarbonat in Pulverform enthält.

6. Vorrichtung zur Durchführung des Verfahrens nach Anspruch 5, die eine erste Leitung (11-15), die mit einem Eingang der Patronen (1-5) verbunden ist, um diesen das Wasser zuzuführen und in ihnen Lösungen zu erzeugen, eine zweite Leitung (21-25), die mit einem Ausgang der Patronen verbunden ist, um die Lösungen einem oberhalb eines Dialysekreislaufs (100) angeordneten Mischungspunkt (10) zuzuführen, ein Meßgerät (210-250) zur Messung der Konzentration der Lösungen und einen Durchsatzregler (110-150) zur Einstellung der Konzentration der Lösungen entsprechend den zumindest vom Meßgerät gelieferten Daten umfaßt, dadurch **gekennzeichnet,** daß das Meßgerät (210-250) in der zweiten Leitung (21-25) oberhalb des Mischungspunktes (10) angebracht ist, um die Konzentration der Lösungen am Ausgang der Patronen zu messen.

7. Vorrichtung nach Anspruch 6, dadurch **gekennzeichnet,** daß sie außerdem noch ein zweites unterhalb des Dialysekreislaufs (100) angebrachtes Meßgerät (101) umfaßt.

8. Vorrichtung nach einem der Ansprüche 6 und 7, dadurch **gekennzeichnet,** daß der Durchsatzregler (110-150) in der ersten Leitung (11-15) oberhalb der Patronen (1-5) angebracht ist, um den Wasserdurchsatz zu regeln und so entsprechend den vom ersten und/oder zweiten Meßgerät gelieferten Daten die Konzentration der Lösungen einzustellen.

9. Vorrichtung nach einem der Ansprüche 6 und 7, dadurch **gekennzeichnet,** daß der Durchsatzregler (110-150) in der zweiten Leitung (21-25) angebracht ist.

10. Vorrichtung nach Anspruch 6, dadurch **gekennzeichnet,** daß jede Patrone ein Mittel zur Verteilung und Umwälzung des Wassers zwischen dem Ein- und Ausgang der Patronen umfaßt.

11. Vorrichtung nach Anspruch 10, dadurch **gekennzeichnet,** daß das Mittel annähernd entlang einer Längsachse der Patronen angeordnet ist.

12. Vorrichtung nach Anspruch 10 oder 11, dadurch **gekennzeichnet,** daß sie aus wenigstens einer semipermeablen Membranfaser (6) besteht.

13. Vorrichtung nach Anspruch 12, dadurch **gekennzeichnet,** daß die Patronen und Fasern so dimensioniert sind, daß die Ionenkonzentrationen in Millimol pro Liter in der erhaltenen Dialyselösung innerhalb der nachfolgenden Bereiche liegen:
- 135 bis 150 mmol für die Natriumionen,
- 1 bis 3 mmol für die Kaliumionen,
- 0 bis 1,75 mmol für die Calciumionen,
- 0,25 bis 1 mmol für die Magnesiumionen,
- 25 bis 35 mmol für die Bicarbonationen.

14. Vorrichtung nach Anspruch 6, dadurch **gekennzeichnet,** daß der Mischungspunkt aus einer dritten, im Eingang des Dialysekreislaufs angebrachten Leitung (10) besteht.

15. Vorrichtung nach Anspruch 6 oder 7, dadurch **gekennzeichnet,** daß die Meßgeräte Leitfähigkeitszellen (210-250) umfassen.

16. Vorrichtung nach Anspruch 8 oder 9, dadurch **gekennzeichnet,** daß der Durchsatzregler Pumpen mit veränderlichem Durchsatz (110-150) umfaßt.

17. Vorrichtung nach Anspruch 14, dadurch **gekennzeichnet,** daß sie ein Mittel zur Messung (200-201) des pH des in der dritten Leitung erhaltenen Gemisches umfaßt.

18. Vorrichtung nach Anspruch 14, dadurch **gekennzeichnet,** daß sie Mittel für die Speisung der dritten Leitung (10) mit Zusätzen wie Glucose, Aminosäuren und Spurenelementen umfaßt.

19. Vorrichtung nach einem der Anspruch 6 bis 18, dadurch **gekennzeichnet,** daß sie Mittel zur Temperaturregelung für das Halten der Dialyselösung auf einer vorgegebenen Temperatur umfaßt.

20. Vorrichtung nach einem der vorhergehenden Ansprüche, dadurch **gekennzeichnet,** daß die Patronen in einem Gehäuse (7) untergebracht sind.
